# EUROPEAN PATENT APPLICATION

(11) **EP 2 949 318 A1**
(43) Date of publication of application: **02.12.2015**
(21) Application number: 15169083.1
(22) Date of filing: 25.05.2015
(51) Int. Cl.: A61K 9/16

(54) **Pharmaceutical compositions comprising racecadotril**

(30) Priority: 26.05.2014 EP 14169873; 26.05.2014 EP 14169874; 26.05.2014 EP 14169865; 26.05.2014 EP 14169868
(71) Applicant: Galenicum Health S.L., 08005 Barcelona (ES)
(72) Inventor: Arroyo Hidalgo, Sergio, 08005 Barcelona (ES); Puigvert Colomer, Marina, 08005 Barcelona (ES)
(74) Representative: Galenicum Health S.L.

(57) **Abstract**

The present invention relates to pharmaceutical compositions comprising the active agent racecadotril, to a process for the preparation thereof, and to the use thereof in the treatment of diarrhoea.

## Description

The present invention relates to pharmaceutical compositions comprising the active agent racecadotril, to a process for the preparation thereof, and to the use thereof in the treatment of diarrhoea.

### STATE OF THE ART

(*RS*)-benzyl N-[3-(acetylthio)-2-benzylpropanoyl]glycinate is also known as benzyl (*RS*)-2-[[2-[(acetylsulphanyl)methyl]-3-phenylpropanoyl]amino]acetate, racecadotril or acetorphan, its empirical formula is C21H23NO4S and the compound has a molecular weight of 385.48 g/mol. Racecadotril is the compound of structure (I) in the form of a racemate:

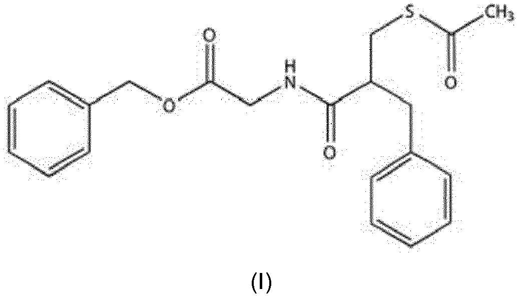

Racecadotril is an antidiarrheal drug which acts as a peripherally acting enkephalinase inhibitor. Unlike other medications used to treat diarrhea, which reduce intestinal motility, racecadotril has an antisecretory effect, reducing the secretion of water and electrolytes into the intestine.

Hidrasec® and Tiorfan® are medicinal products indicated for the symptomatic treatment of acute diarrhoea in adults when causal treatment is not possible. If causal treatment is possible, racecadotril can be administered as a complementary treatment. These products contain racecadotril as the active agent and are presented as a granulate for oral suspension containing 10 or 30 mg of the active agent.

Racecadotril was first disclosed in EP0038758. WO01/97803, EP1294372, US2009/0186084 and EP2462922 disclose oral formulations comprising said active agent.

### DESCRIPTION OF THE INVENTION

The present invention provides a stable pharmaceutical composition in the form of a powder comprising racecadotril as active agent which has a low impurity content and can be easily manufactured and filled into sachets or sticks. Moreover, said compositions show a good stability. Surprisingly, said compositions have a good taste, although the active agent has a bitter taste and sulfur odor, the powder compositions of the present invention mask this unpleasant taste.

The inventors have surprisingly found that stable powder compositions comprising racecadotril can be produced either by a simple direct mix of the active agent and the excipients, or by a dry granulation of the active agent and at least one diluent wherein the weight ratio active agent:diluent in the granulate is between 1:5 and 1:1000. Said compositions overcome the problem of the bitter taste and bad flavour of the active agent racecadotril. Importantly, these pharmaceutical compositions do not need to be coated or encapsulated, because they have a good taste in mouth in order to prepare a composition which tastes good when taken with water or with food. Thus, the compositions of the present invention are easy to take even by children.

Moreover, in the case of the direct mix, the process of manufacture is faster and simpler than those of the prior art, which comprise the granulation of the active agent.

In a first aspect, the present invention relates to a pharmaceutical composition comprising the active agent racecadotril, wherein said pharmaceutical composition is in the form of a pharmaceutical powder consisting of a direct mix comprising the active agent racecadotril and at least one diluent; or wherein said pharmaceutical composition comprises a compacted granulate comprising the active agent racecadotril and at least one diluent, wherein the weight ratio active agent:diluent in the pharmaceutical composition is between 1:5 and 1:1000.

Thus, in a first aspect, the present invention relates to a pharmaceutical composition in the form of a pharmaceutical powder consisting of a direct mix comprising the active agent racecadotril and at least one diluent. Also, in a first aspect, the present invention relates to a pharmaceutical composition comprising a compacted granulate comprising the active agent racecadotril and at least one diluent, wherein the weight ratio active agent:diluent in the pharmaceutical composition is between 1:5 and 1:1000.

The term "pharmaceutical composition" as used herein refers to a finished dosage formulation, which contains the active agent racecadotril and which is in the form in which it can be marketed for use.

The term "pharmaceutical powder" as used herein refers to an intimate mixture of dry particles of an active agent and at least one pharmaceutically acceptable excipient or inactive ingredient.

The term "direct mix" as used herein refers to a product which simply consists of a mixture of different components.

The term "pharmaceutical granulate" or "granulate" or "granular component" as used herein refers to the part of the pharmaceutical composition that has been obtained by granulating a powder into larger particles herein called granules. As used herein, the term "compacted granulate" refers to a granulate that has been obtained by dry granulation, i.e: by compacting a pharmaceutical powder and then milling and sieving the slug or laminate obtained by compaction. The dry granulation is performed for example by slugging or using a roller compactor.

In a preferred embodiment of the first aspect, the weight ratio active agent:diluent is between 1:10 and 1:1000, preferably between 1:50 and 1:200, more preferably between 1:75 and 1:150.

The term "diluent" as used herein refers to pharmaceutically acceptable excipients which are added to the bulk volume of the active agent making up the solid composition. As a result, the size of the solid composition increases, which makes its size suitable for handling. Diluents are convenient when the dose of drug per solid composition is low and the solid composition would otherwise be too small.

In a preferred embodiment, the particle size volume distribution of the active agent has a D50 between 1 and 10 microns and/or a D90 between 12 and 40 microns, when measured by laser diffraction analysis. In a preferred embodiment, the particle size volume distribution of the active agent has a D50 between 2 and 9 microns and/or a D90 between 14 and 35 microns, when measured by laser diffraction analysis. In a preferred embodiment, the particle size volume distribution of the active agent has a D50 between 3 and 8 microns and/or a D90 between 16 and 30 microns, when measured by laser diffraction analysis.

In a preferred embodiment, said pharmaceutical composition is free of a disintegrant. As used herein, "disintegrant" means a pharmaceutically acceptable excipient or a mixture of pharmaceutically acceptable excipients added to a formulation to facilitate its breakup or disintegration after administration.

The term "pharmaceutically acceptable" indicates that the substance or composition must be compatible chemically and/or toxicologically, with the other ingredients comprising a formulation, the mammal being treated therewith, and/or the route of administration of the composition.

In a preferred embodiment, said pharmaceutical composition is free of a polymethacrylate.

In a preferred embodiment, the pharmaceutical composition of the present invention is stable. The term "stable" as used herein refers to a pharmaceutical composition comprising racecadotril wherein the total content of impurities originating from the decomposition of racecadotril does not exceed 5 % area, preferably 3 % area, more preferably 2 % area and most preferably 1 % area determined by liquid chromatography (HPLC) at 230 nm if such a composition is stored for 1 month at 40 °C and 75 % relative humidity (RH).

In a preferred embodiment, said pharmaceutical composition is free of magnesium stearate.

In a preferred embodiment, the diluent is a soluble powder. In a preferred embodiment, the diluent is a water soluble powder.

In a preferred embodiment, the diluent is a sugar or calcium phosphate or a mixture thereof. Preferably, the diluent is a sugar selected from sucrose, xylitol, mannitol, sorbitol, maltose, isomaltose, dextrose, fructose and mixtures thereof. More preferably, the diluent is sucrose.

In a preferred embodiment of the first aspect, the pharmaceutical composition further comprises a flavoring agent.

In another preferred embodiment of the first aspect, the pharmaceutical composition further comprises a sweetening agent. The term "sweetening agent" as used herein includes compounds capable of enhancing or intensifying the perception of sweet taste of sweetener compositions or sweetened compositions. The term sweetening agent is synonymous with the terms "sweetness enhancer", "sweet taste potentiator", "sweetness potentiator", and "sweetness intensifier".

In a preferred embodiment, the sweetening agent is selected from sucralose, acesulfame, aspartame, cyclamate, neohesperidin dihydrochalcone, saccharin and mixtures thereof. Preferably, the sweetening agent is sucralose.

In another preferred embodiment of the first aspect, the pharmaceutical composition further comprises at least an acid. Preferably, the acid or acids are selected from citric acid, tartaric acid, ascorbic acid and mixtures thereof. More preferably, the acid is citric acid, preferably anhydrous citric acid.

In a preferred embodiment, the pharmaceutical composition comprises from 5 to 40 mg of active agent per unit dose. Preferably, the pharmaceutical composition comprises from 9 to 11 mg or from 28 to 32 mg of active agent per unit dose. As used herein, the term "unit dose" or "unit dosage" refers to a physically discrete unit that contains a predetermined quantity of active agent calculated to produce a desired therapeutic effect. The unit dose or unit dosage may be in the form of a vial, tablet, capsule, sachet, etc. referred to herein as a "unit dosage form".

In a preferred embodiment, the pharmaceutical composition is packaged in a sachet or stick pack. Preferably, the sachet or stick pack consists essentially of printed paper foil/aluminium foil/polyethylene foil.

The term "sachet" or "stick pack" as used herein refers to a small, sealed packet containing a quantity of material, which is a single use or unit dose quantity. A packaging of the stick pack type, preferably with improved opening, comprises normally: a flexible film, having at least one layer, which forms a hermetically sealed tubular body with mutually opposite longitudinal film flaps, a first band provided longitudinally to said body for inside/outside sealing of said mutually opposite longitudinal flaps of the film; second sealing bands provided transversely to said body for inside/outside sealing; a sealed extension region protruding from at least one of said second sealing bands on a respective portion of at least one edge of said tubular body; and preferably a transverse pre-weakening incisions that are provided in longitudinal alignment with said sealed extension region, along at least one of said mutually opposite longitudinal flaps. Suitable stick packs are described in WO9501921.

In a preferred embodiment, the stick pack comprises three layers which are essentially a layer of printed paper foil, a layer of aluminium foil and a layer of polyethylene foil, being the paper layer the external layer and the polyethylene layer, the internal layer.

A second aspect of the present invention relates to a process for the manufacture of the pharmaceutical composition of the first aspect, comprising the following steps:
i) steps of weighing, sieving and mixing the active agent racecadotril and at least one diluent, and, when necessary,
ii) compacting and milling the mix obtained in step (i) to obtain a granulate.

Thus, a second aspect of the present invention relates to a process for the manufacture of the pharmaceutical composition of the first aspect, comprising the steps of weighing, sieving and mixing the active agent racecadotril and at least one diluent. Also, the second aspect of the present invention relates to a process for the manufacture of the pharmaceutical composition of the first aspect, comprising the following steps:
i) weighing, sieving and mixing the active agent racecadotril and at least one diluent; and
ii) compacting and milling the mix obtained in step (i) to obtain a granulate.

A third aspect of the present invention relates to the pharmaceutical composition obtained by the process of the second aspect.

In a fourth aspect, the present invention relates to a pharmaceutical batch comprising at least 20,000 units of the pharmaceutical composition of the first or third aspect. In a preferred embodiment of the fourth aspect, the pharmaceutical batch comprises at least 50,000 units.

The term "batch" as used herein refers to a specific quantity of a drug or other material that is intended to have uniform character and quality, within specified limits, and is produced according to a single manufacturing order during the same cycle of manufacture. A batch, in the case of a drug product produced by continuous process, is a specific identified amount produced in a unit of time or quantity in a manner that assures its having uniform character and quality within specified limits (Code of Federal Regulations Title 21, Food and Drug Administration department of Health and Human Services, Subchapter C, Section 210.3 (b) (2) and (10)).

The term "pharmaceutical batch" as used herein refers to a batch as defined above of a pharmaceutical composition manufactured in accordance with the principles and guidelines of Good Manufacturing Practice (GMP) at an industrial scale and which is intended for commercialization (Directive 91/356/EEC).

The pharmaceutical composition may be manufactured at laboratory scale, not necessarily following GMP and not intended for commercialization. The pharmaceutical composition may also be manufactured for validation, following GMP. A batch of a pharmaceutical composition which is manufactured for validation is called "pilot batch".

Each pharmaceutical batch of finished product must fulfil the regulatory requirements of the corresponding Medicine Agency before being released for sale or supply, such as impurities thresholds and stability data.

The term "uniform" as used herein refers to the content of the active agent in the compositions of a pharmaceutical batch has to be homogeneous. According to the FDA criteria, uniformity is considered as achieving 90-110 % potency of the theoretical strength with a relative standard deviation (RSD) of less than 5 % for all samples (Guidance for Industry ANDA's: Blend Uniformity Analysis, published August 1999).

The term "active agent" refers to a therapeutically active compound, as well as any prodrugs thereof and pharmaceutically acceptable salts, hydrates and solvates of the compound and the prodrugs.

For the release of a pharmaceutical batch the distribution of the active agent in the pharmaceutical compositions has to be homogeneous, that is, content uniformity is required. All batches are expected to be uniform within normal process variation. Process validation studies are conducted prior to the marketing of a drug product to assure that production processes are controlled. The test batch is manufactured prior to validation, yet it is the basis on which an application is approved (MANUAL OF POLICIES AND PROCEDURES, MAPP 5225.1).

It is essential, therefore, to assure that the test batch is uniform. In-process tests for uniformity should be conducted throughout the entire production process, e.g., at commencement or completion of significant phases (21 CFR 211.110). These tests should be designed to detect potential in-process anomalies (MAPP 5225.1).

A fifth aspect of the present invention relates to the pharmaceutical composition of the first or third aspect or the pharmaceutical batch of the fourth aspect, for use in the treatment of diarrhoea, preferably acute diarrhoea in infants.

A sixth aspect of the present invention relates to a cardboard box with a patient information leaflet comprising at least one unit of the pharmaceutical composition of the first or third aspect.

A seventh aspect of the present invention relates to a method for preparing a pharmaceutical dossier to obtain the marketing authorization of pharmaceutical composition of the first or third aspect comprising the following steps:
i) manufacturing at least one pharmaceutical batch of the fourth aspect;
ii) performing stability tests of the batches of step (i);
iii) compiling the results obtained in steps (i) to (iii); and
iv) providing the compiled results of step (iii) in a data carrier.

The term "pharmaceutical dossier" to obtain the marketing authorization refers to a dossier with data proving that the drug has quality, efficacy and safety properties suitable for the intended use, additional administrative documents, samples of finished product or related substances and reagents necessary to perform analyzes of finished product as described in that dossier.

In an eighth aspect, the present invention relates to a data carrier comprising the compiled results of a pharmaceutical dossier obtained by the method of the seventh aspect. In a preferred embodiment of the eighth aspect, said data carrier is selected from the group consisting of a digital data carrier such as CD, DVD, USB, hard drive; and paper.

The term "data carrier" refers to a device for recording (storing) information (data). The recording can be done using virtually any form of energy. A storage device may hold information, process information, or both. Most often the term is used with computers. Data carrier can permanently hold data, like files.

All percentages, parts, and ratios herein are by weight unless specifically noted otherwise. As used herein, the term "about" refers preferably to a range that is ±10 %, preferably ±5 %, or more preferably ±1 % of a value with which the term is associated.

Unless otherwise indicated, all the analysis methods are carried out according to the European Pharmacopoeia 7th edition.

### EXAMPLES

The following examples illustrate various embodiments of the invention and are not intended to limit the invention in any way:

### Example 1. Pharmaceutical compositions.

| | Ex. 1a (mg) | Ex. 1b (mg) | Ex. 1c (mg) | Ex. 1d (mg) |
|---|---|---|---|---|
| Active agent | 30 | 30 | 30 | 30 |
| Sucrose | 2904 | 2974 | 2971 | 1907 |
| Isomaltose | - | - | - | 964 |
| Anhydrous Colloidal Silica | 6 | 6 | 6 | 6 |
| Flavour | 60 | 60 | 60 | 60 |
| Anhydrous citric acid | - | 30 | 30 | 30 |
| Sucralose | - | - | 3 | 3 |

For examples 1a to 1c, first, the active agent, half of the sucrose, the anhydrous colloidal silica and optionally the anhydrous citric acid were added to a mixer and blended at 300 rpm for 5 minutes. Then, the rest of the components were added and blended at 600 rpm for 15 minutes. Finally, sachets or stick packs were filled with the amount corresponding to an active agent unit dose of 10 mg or 30 mg.

For example 1d, first the active agent, the isomaltose, the anhydrous colloidal silica and the anhydrous citric acid were sieved and mixed. Then, the sucrose, the sucralose and the flavour were sieved and mixed with the rest of the components. Finally, sachets or stick packs were filled with the amount corresponding to an active agent unit dose of 10 mg or 30 mg.

### Example 2. Organoleptic analysis of the pharmaceutical compositions.

The pharmaceutical compositions of example 1 were prepared and tasted in a double blind process, where the product Tiorfan® was included. The powder was placed in the mouth and the taste was evaluated in the following scale: --/-/o/+/++, being -- a bad taste and ++ a good taste. The following table illustrates the results of the organoleptic analysis:

| Tiorfan® granules for oral suspension | ○ |
|---|---|
| Example 1a | ○ |
| Example 1b | + |
| Example 1c | ++ |

### Example 3. Particle size volume distribution.

The particle size distribution of the active agent was analysed by laser diffraction spectroscopy using a Malvern Mastersizer 2000 particle size analyzer.

### Example 4. Pharmaceutical compositions.

| | Ex. 4a (mg) | Ex. 4b (mg) |
|---|---|---|
| *Intragranular* | | |
| Active agent | 30 | 30 |
| Sucrose | 2,904 | 290 |
| Flavour | 60 | - |
| Anhydrous Colloidal Silica | 6 | 6 |

| *Extragranular* | | |
|---|---|---|
| Sucrose | - | 2,614 |
| Flavour | - | 60 |

Example 4a: the active agent, half of the sucrose and the anhydrous colloidal silica were blended. Then, the rest of the components were added and blended. This blend was compacted and milled, and sachets or stick packs were filled with the resultant granulate in an amount corresponding to an active agent unit dose of 10 mg or 30 mg.

Example 4b: the active agent, the anhydrous colloidal silica and 10 % of the sucrose were blended. This blend was compacted and milled to give a granulate. Then, the rest of the components were added to the granulate and blended, and sachets or stick packs were filled with this final blend in an amount corresponding to an active agent unit dose of 10 mg or 30 mg.

## Claims

1. A pharmaceutical composition comprising the active agent racecadotril, wherein said pharmaceutical composition is in the form of a pharmaceutical powder consisting of a direct mix comprising the active agent racecadotril and at least one diluent; or wherein said pharmaceutical composition comprises a compacted granulate comprising the active agent racecadotril and at least one diluent, wherein the weight ratio active agent:diluent in the pharmaceutical composition is between 1:5 and 1:1000.

2. The pharmaceutical composition according to the preceding claim, wherein the weight ratio active agent:diluent is between 1:10 and 1:1000, preferably between 1:50 and 1:200, more preferably between 1:75 and 1:150.

3. The pharmaceutical composition according to any one of the preceding claims, wherein said pharmaceutical composition is free of a disintegrant, free of a polymethacrylate and/or free of magnesium stearate.

4. The pharmaceutical composition according to any one of the preceding claims, wherein the diluent is a soluble powder, preferably the diluent is a sugar or calcium phosphate or a mixture thereof.

5. The pharmaceutical composition according to any one of the preceding claims, wherein the diluent is a sugar selected from sucrose, xylitol, mannitol, sorbitol, maltose, isomaltose, dextrose, fructose and mixtures thereof.

6. The pharmaceutical composition according to any one of the preceding claims, further comprising: (a) a flavoring agent; and/or (b) a sweetening agent, preferably selected from sucralose, acesulfame, aspartame, cyclamate, neohesperidin dihydrochalcone, saccharin and mixtures thereof, more preferably the sweetening agent is sucralose; and/or (c) at least an acid, preferably selected from citric acid, tartaric acid, ascorbic acid and mixtures thereof, more preferably the acid is anhydrous citric acid.

7. The pharmaceutical composition according to any one of the preceding claims, wherein the pharmaceutical composition comprises from 5 to 40 mg of active agent per unit dose, preferably from 9 to 11 mg or from 28 to 32 mg of active agent per unit dose.

8. The pharmaceutical composition according to any one of the preceding claims, wherein the pharmaceutical composition is packaged in a sachet or stick pack, preferably the sachet or stick pack consists essentially of printed paper foil/aluminium foil/polyethylene foil.

9. A process for the manufacture of the pharmaceutical composition as defined in any one of the preceding claims, comprising the following steps:
i) weighing, sieving and mixing the active agent racecadotril and at least one diluent, and, when necessary,
ii) compacting and milling the mix obtained in step (i) to obtain a granulate.

10. The pharmaceutical composition obtained by the process of the preceding claim.

11. A pharmaceutical batch comprising at least 20,000 units, preferably at least 50,000 units, of the pharmaceutical composition as defined in any one of claims 1 to 8 or 10.

12. The pharmaceutical composition according to any one of claims 1 to 8 or 10 or the pharmaceutical batch according to claim 11, for use in the treatment of diarrhoea, preferably acute diarrhoea in infants.

13. A cardboard box with a patient information leaflet comprising at least one unit of the pharmaceutical composition as defined in any one of claims 1 to 8 or 10.

14. A method for preparing a pharmaceutical dossier to obtain the marketing authorization of pharmaceutical composition as defined in any one of claims 1 to 8 or in claim 10 comprising the following steps:
i) manufacturing at least one pharmaceutical batch as defined in claim 11;
ii) performing stability tests of the batches of step (i);
iii) compiling the results obtained in steps (i) to (iii); and
iv) providing the compiled results of step (iii) in a data carrier.

15. A data carrier comprising the compiled results of a pharmaceutical dossier obtained by the method of the preceding claim, preferably, wherein said data carrier is selected from the group consisting of a digital data carrier such as CD, DVD, USB, hard drive; and paper.
